Europäisches Patentamt

European Patent Office

Office européen des brevets

(19)

(11) **EP 1 080 109 B1**

(12) **EUROPEAN PATENT SPECIFICATION**

(45) Date of publication and mention
of the grant of the patent:
**09.07.2003 Bulletin 2003/28**

(21) Application number: **99926409.6**

(22) Date of filing: **25.05.1999**

(51) Int Cl.[7]: **C07K 14/20**, C07K 1/36

(86) International application number:
**PCT/EP99/03602**

(87) International publication number:
**WO 99/061473 (02.12.1999 Gazette 1999/48)**

(54) **METHOD FOR PURIFICATION OF BORRELIA LIPOPROTEIN OSPA**

VERFAHREN ZUR AUFREINIGUNG DES BORRELIA LIPOPROTEINS OSPA

PROCEDE DE PURIFICATION DE LA LIPOPROTEINE OSPA BORRELIA

(84) Designated Contracting States:
**AT BE CH CY DE DK ES FI FR GB GR IE IT LI LU
MC NL PT SE**
Designated Extension States:
**SI**

(30) Priority: **26.05.1998 GB 9811219**

(43) Date of publication of application:
**07.03.2001 Bulletin 2001/10**

(73) Proprietor: **GlaxoSmithKline Biologicals S.A.
1330 Rixensart (BE)**

(72) Inventors:
• **DESMONS, Pierre
  1330 Rixensart (BE)**
• **LIVEYNS, Robert
  1330 Rixensart (BE)**
• **MERTENS, Emmanuel
  1330 Rixensart (BE)**
• **CHAMPLUVIER, Benoit
  1330 Rixensart (BE)**
• **PLAINCHAMP, Dominique
  1330 Rixensart (BE)**
• **CORAZZA, Yvon
  1330 Rixensart (BE)**

(74) Representative: **Privett, Kathryn Louise et al
GlaxoSmithKline
Corporate Intellectual Property CN9.25.1)
980 Great West Road
Brentford, Middlesex TW8 9GS (GB)**

(56) References cited:
**EP-A- 0 522 560          WO-A-90/04411
WO-A-93/08299**

• CALDWELL S R ET AL: "Large scale purification
  process for recombinant NS1- OspA as a
  candidate vaccine for Lyme disease."
  BIOSEPARATION, (1996 APR) 6 (2) 115-23. ,
  XP002116461
• JIANG W ET AL: "Purification of Borrelia
  burgdorferi outer surface protein A ( OspA ) and
  analysis of antibody binding domains."
  CLINICAL AND DIAGNOSTIC LABORATORY
  IMMUNOLOGY, (1994 JUL) 1 (4) 406-12. ,
  XP002116462
• GONDOLF K B ET AL: "ISOLATION OF AN
  OUTER MEMBRANE PROTEIN COMPLEX FROM
  BORRELIA BURGDORFERI BY N-BUTANOL
  EXTRACTION AND HIGH-PERFORMANCE ION
  -EXCHANGE CHROMATOGRAPHY" JOURNAL
  OF CHROMATOGRAPHY, vol. 521, no. 2, 23
  November 1990 (1990-11-23), pages 325-334,
  XP002041529 ISSN: 0021-9673

## Description

### Field of the Invention:

The present invention relates to an improved purification of *Borrelia* lipoprotein OspA.

### Background of the Invention:

Lyme disease in humans is a chronic progressive disease caused by *B. burgdorferi,* which is transmitted to humans mainly by *Ixodes* ticks. The disease attacks many organs, notably the skin, heart, liver, central and peripheral nervous system, kidneys as well as the musculoskeletal system.

*Borrelia burgdorferi* (sensu lato) is a generic term which encompasses several Borrelia species which are believed to be the causative agent of Lyme borreliosis (Lyme disease). At a minimum, this includes: *B. burgdorferi* sensu stricto, *B. garinii*, and *B. afzelii*. This disease is transmitted by the bite of various species of *Ixodes* ticks carrying the spirochete. The main reservoir of the infection in the United States is the white-footed mouse and the infection can be transmitted to many mammalian species including man.

OspA is a protective antigen of the Lyme disease spirochete *Borrelia burgdorferi* sensu lato. Expression of the full length OspA gene in *Borrelia* or *E. coli* results in a protein that is processed posttranslationally by signal peptidase II and contains an attached lipid moiety. As will be seen herein, it has now been shown that purification of lipoprotein OspA on an industrial scale, which is suitable for clinical use, can be carried out without requiring heating or alcohol extraction in the purification procedure, and yields an OspA lipoprotein that is essentially free from detergent.

### Summary of the Invention:

In one aspect, the present invention provides a method for purification of *Borrelia* lipoprotein OspA suitable for human clinical use, which comprises (a) lysis of a host cell expressing lipoprotein OspA in the presence of a zwitterionic detergent to form an impure solution containing lipoprotein OspA; (b) clarifying the impure solution containing lipoprotein OspA to remove host cell debris; (c) contacting the impure solution with an anion exchange resin in the presence of a nonionic detergent; (d) collecting the flow through from said anion exchange resin and contacting said flow through with a cation exchange resin; and (e) eluting OspA followed by a gel filtration step, wherein the purification process occurs without heating.

In a related aspect, the present invention provides a method for purification of *Borrelia* lipoprotein OspA that is detergent free.

In yet another related aspect, the present invention provides pure and stable lipoprotein OspA that is detergent free and suitable for human clinical use.

### Brief Description of the Drawings:

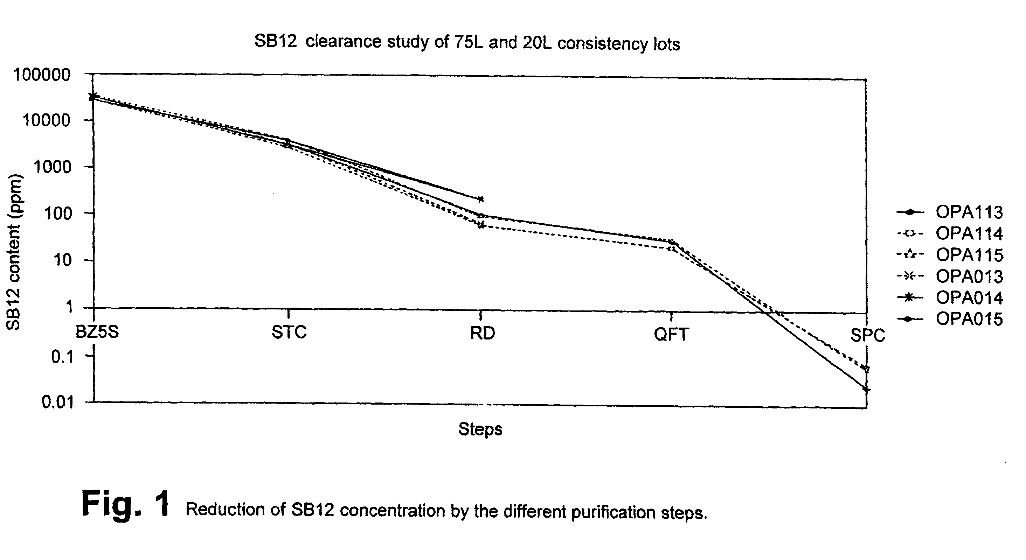

Figure 1 shows the reduction in SB12 concentration at different purification steps of Example 1. Three 75 L batches (OPA113, OPA114 and OPA115) and three 20 L batches (OPA013, OPA014 and OPA015) were analyzed. The amount of SB12 was obtained from a calibration curve taking into account the corresponding dilution factor. BZSS = cell homogenate; STC = elution pool of Streamline SP column; RD = retentate of diafiltration; QFT = flow through of Q-Sepharose FF column; SPC = flow through of SP-Sepharose FF column.

### Detailed Description of the Invention:

The present invention provides a simple method for scaling up purification of *B. burgdorferi* lipoprotein OspA from the lab bench scale to an industrial scale. The method has the advantages in that it does not require heating or alcohol or biphasic extraction for purification, and yields a high degree of purity within a single step. The method of the present invention results in an OspA protein suitable for human clinical use. The present invention also provides OspA lipoprotein that is essentially free from detergent (i.e., only a trace amount remains) and is stable for prolonged periods of time (2 years in PBS @ 2-8°C).

The present invention provides a method for purification of lipoprotein OspA, in the presence of a zwitterionic detergent to solubilize OspA, without the need for heating or alcohol extraction in the purification process. Advantageously, this avoids potential impairments to the immunogenicity of the final product (e.g., denaturation, delipidation, etc). Once the OspA lipoprotein is solubilized it is preferable to continue purification in the presence of a detergent, even though detergent is not desirable in the final product, as the lipoprotein tends to form lipid aggregates during the purification process in the absence of detergent. Preferably another detergent is added during the ion-exchange purification to keep lipoprotein OspA in solution and in a non-aggregated random state. Such detergent is preferably a nonionic detergent, such as Triton™ (polyoxyethylene ether) or Tween™ (polyoxyethylenesorbitan ester). Once purified, lipoprotein OspA is stable in PBS (phosphate buffered saline) in the absence of detergent (surfactant). Purified lipoprotein OspA does not require detergent in the final product, as once it is pure it is also stable in a micellular-like form.

Advantageously, the present invention provides a method to purify lipoprotein OspA from *Borrelia burgdorferi* sensu lato strains. Such strains include *Borrelia burgdorferi* sensu stricto (e.g., ZS7, B31, N40, JD1, 297, Sh-2-82, etc), *Borrelia garinii* (e.g. ZQ1, IP90, NE11H, IP3, etc.) and *Borrelia afzelli* (e.g., ACA-1, PKo, Bo23, etc.).

The purification scheme of the present invention comprises the following steps. Cell lysis of a host cell expressing lipoprotein OspA in the presence of a zwitterionic detergent whereby lipoprotein OspA is solubilized; a clarification step to remove host cell debris comprising a fluidized bed ion exchange followed by diafiltration (or alternatively centrifugation); optionally a filtration step to remove any remaining particulate matter; an anion exchange; a cation exchange; and a gel filtration (sizing column) to remove any non-lipidated OspA from lipidated OspA. In addition, it is preferable to add a sterile filtration step after the gel filtration.

The purification of lipoprotein OspA is further illustrated in the Examples section. Scheme I involves four chromatographic steps and one diafiltration. That process involves: cell homogenization (disruption), for example, by a high pressure cell homogenizer; dilution of the homogenate (for example, to an approximate optical density (OD) of 20) and acidification of the homogenate; followed by a cation exchange on a fluidized bed (sometimes referred to as expanded bed adsorption - for example, the Streamline™ SP system, Phannacia). The homogenate is loaded on the fluidized bed, washed and eluted by increasing the ionic strength and/or increasing the pH. The eluant is optionally sterile filtered and then diafiltered. The sample containing lipoprotein OspA is then loaded onto an anion exchange resin. The flow-through is loaded onto another cation exchange resin. The resin is washed and OspA is eluted by increasing the ionic strength and/or increasing the pH. The OspA in solution is passed through a sizing column and then sterile filtered.

The expanded bed adsorption is based on fluidization. In practice, this means that adsorbent particles are raised by the upward liquid flow to balance the downward force of gravity. A stable homogeneous expanded bed is created by lifting the particles just enough to suspend them in the liquid flow. In one embodiment, it uses an adsorption called Streamline™ (a sulfopropyl derivatized resin for cation exchange).

Diafiltration is used to eliminate the zwitterionic detergent and to concentrate the OspA protein. The retentate is applied on an anion exchange column (e.g., Q-Sepharose) in the presence of a nonionic detergent (e.g., Triton™ X-100). More than 95% of contaminants are removed after the anion exchange step. Lipoprotein-OspA is not bound to the column and the flow through is injected onto a cation exchange (e.g., SP Sepharose) column. The column is washed and lipoprotein-OspA is eluted to be injected on a gel permeation column (e.g., Sephacryl S300 HR) to exchange buffer, separate non-lipidated OspA from lipidated OspA and eliminate the detergent. The purified lipoprotein-OspA is diluted to a standard concentration and sterilized by validated filtration on a 0.2 um sterile membrane.

Ion exchange resins are well known in the art. Cation exchangers include CM Sephadex, SP Sephadex, CM Sepharose, S Sepharose, CM cellulose, etc. A preferred resin for cation exchange chromatography is SP or CM Sepharose (Pharmacia). Anion exchangers include DEAE Sephadex, QAE Sephadex, DEAE Sepharose, Q Sepharose, DEAE Sephacel, etc. A preferred resin for anion exchange chromatography is Q Sepharose (Pharmacia). Gel filtration (sizing) resins include Sephadex, Sephacryl, Sepharose, Superdex, Superose, etc. A preferred resin step is Sephacryl S resin, such as S-300 HR (Pharmacia).

The resulting purified OspA protein is free from detergents or surfactants.

Scheme II involves three purification steps. Cell lysis (e.g., freeze-thaw) in the presence of a zwitterionic detergent; centrifugation and an optional filtration of the supernatant (impure solution) containing OspA. When conducting the optional filtration step, it is preferable to use prefilters, e.g., 1.2um filter followed by 0.45um filter and then a .22um filtration. The filtrate containing lipoprotein OspA is then loaded onto an anion exchange resin in the presence of a nonionic detergent. The flow-through is loaded onto a cation exchange resin. The resin is washed and OspA is eluted by increasing the ionic strength and/or increasing the pH. The OspA in solution is passed through a sizing column and then sterile filtered.

Under Scheme II, cell lysis occurs automatically in the presence of zwitterionic detergent. There is no need for cell disruption. The diafiltration step is not necessary. As in Scheme I, more than 95% of contaminants are removed after the anion exchange step. OspA does not bind to Q Sepharose under basic conditions.

Once purified in the absence of detergent (surfactant), lipoprotein OspA is pure and stable in PBS (phosphate buffered saline).

Zwitterionic detergents include the N-Alkyl-N,N-dimethylammonio-1-propanesulfonates (i.e., SB 8, 10, 12, 14, 16, 18), CHAPS (3-[(3-Cholamidopropyl)-dimethylammonio]-1-propanesulfonate) and CHAPSO (3-[(3-Cholamidopropyl)dimethylammonio]-2-hydroxy-1-propanesulfonate). Preferably, the zwitterionic detergent is selected from the N-Alkyl-N,N-dimethylammonio-1-propanesulfonates. More preferably, it is N-Dodecyl-N,N-dimethyl-3-ammonio-1-propanesulfonate (referred to as SB12, also known as lauryl-sulfobetain). SB12 does not form two phases (biphasic) in an aqueous solution, and is thus easier for scaling up the purification of OspA.

By following the purification scheme of the present invention, the level of SB12 in a purified lipoprotein OspA solution (1mg/ml) is less than 1000 ppb [part per billion] (1ppm). Preferably it is less than 100 ppb (0.1ppm). More preferably, it is less than 10 ppb (0.01ppm) which is approaching the level of detection.

Nonionic detergents include Triton™ (polyoxyethylene ethers), Tween™ (polyoxyethylenesorbitan esters), polyoxyethylene ester, and Nonidet P-40. Preferably, it is Triton™ (X-100, X-114, X-405, N-101) or Tween™ (20-80). More preferably it is Triton™ X-100 or Triton™ X-114. As Triton™ X-100 is known to be safe and approved by FDA, it is the most preferred detergent.

By following the purification scheme of the present invention, the level of Triton™ X-100 in a purified lipoprotein OspA solution (1mg/ml) is less than 1000 ppm [part per million] (0.1%). Preferably it is less than 100 ppm. More preferably, it is less than 10 ppm. For a 30ug dose of lipoprotein OspA, the level of Triton™ X-100 per dose is less than 300ng/dose.

Purified lipoprotein OspA of the present invention is detergent free, suitable for human clinical use and is stable. By "purified lipoprotein OspA" is meant greater than 95% purity as determined by SDS-PAGE (Coomassie stained); preferably it is greater than 98% purity.

By "detergent free" is meant that the level of detergent in a purified lipoprotein OspA solution (1mg/ml) is less than 1000 ppm [part per million] (0.1%). Preferably it is less than 100 ppm. More preferably, it is less than 10 ppm. Hence, for a 30ug dose of lipoprotein OspA, the level of detergent (nonionic or zwitterionic) per dose is less than 300ng/dose.

By "suitable for human clinical use" is meant that the endotoxin content for 30ug of lipoprotein OspA is less than 5 EU, as determined by the chromogenic LAL test. In addition, the level of DNA per 30ug of lipoprotein OspA is less than 100pg; preferably it is less than 20pg; more preferably it is less than 2pg. Furthermore, the level of *E. coli* contaminants per 30ug of lipoprotein OspA is less than 0.100% of the total protein content; preferably it is less than 0.050%; more preferably it is less than 0.025%.

By "stable" is meant that lipoprotein OspA does not appreciably degrade, i.e., after one year stored at 2-8°C, 95% or more of OspA remains as a single band as determined by SDS-PAGE (preferably it is 98% or greater) and/or it retains a ratio of lipid to protein of approximately 2 (2.0 +/0.5) and/or it retains 80% or more of its immunogenicity in Balb/C mice (preferably it is 90% or greater, more preferably it is 95% or more).

Purification of lipidated OspA according to the present invention can be performed on the natural *Borrelia burgdorferi* sensu lato strains expressing OspA, or via suitable recombinant expression systems known in the art. For example, a recombinant molecule or vector is constructed in which the full length OspA coding region is operably linked to a heterologous expression control sequence permitting expression of the protein. Methods for obtaining such expression vectors are well known. See, Sambrook et al., "Molecular Cloning. A Laboratory Manual", 2d edition, Cold Spring Harbor Laboratory, New York (1989).

Suitable host cells or cell lines for transfection include bacterial cells. For example various strains of *E. coli* (e.g., AR58, AR68, HB101, MC1061, etc.) are well-known in the art as suitable host cells. Various strains of *B. subtilis, Streptomyces,* and other bacilli can also be used in this method.

In order that this invention may be better understood, the following examples are set forth. These examples are for purposes of illustration only, and are not to be construed as limiting the scope of the invention in any manner.

**Examples:**

**Example 1: Purification of Lipoprotein OspA - Scheme I:**

**Fermentation:**

Full-length OspA was expressed in *E. coli* (strain AR58, see Strickler et al., Proteins, 6:139-154 (1989)), which had been transformed with pOA15 (an *E. coli* plasmid expression vector having the regulatorylexpression elements of pAS1 (Young et al., PNAS, 80: 6105-6109 (1983)) and the kanamycin resistance gene) carrying the coding sequence for *Borrelia burgdorferi* sensu stricto strain ZS7 lipoprotein OspA (see US Patent 5,434,077) under the control of the heat inducible lambda pL promoter. Induction was at 39.5°C +/- 2.0°C for approximately 23 hours.

The fermentation broth was clarified by a tubular bowl (Sharpless) centrifuge. The pellet is recovered from the bowl and frozen at -70°C. The pellet may be stored at -70°C for up to 12 months prior to further processing.

**Extraction:**

Cells are thawed and resuspended in a cell disruption buffer containing SB12 detergent (TBRO buffer containing: 25mM acetate, 4 mM EDTA, pH 6.0, 3% SB12) to an equivalent of $OD_{650}$ = 60 (theoretical value calculated

from the $OD_{650}$ obtained at the end of fermentation - equivalent to 30g dry weight /liter). The cells are disrupted mechanically by passage through a high pressure cell homogenizer (Rannie). The cell homogenate is diluted three times (to $OD_{650}$ = 20) with TBDI buffer (25mM acetate, 4 mM EDTA, pH 4.8, 3% SB12) and the diluted homogenate is left standing for 1 hr. The pH is adjusted to 4.8 ± 0.1 with acetic acid and conductivity is adjusted to 3.8 mS ± 0.2 mS. (All conductivity values are converted to 20°C) If required NaCl can be added to increase conductivity or $H_2O$ added to decrease conductivity.

**Purification:**

Purification is achieved by four chromatographic steps and one diafiltration step. First, lipoprotein-OspA is adsorbed on a Streamline SP matrix in an expanded bed column. The gel is washed to eliminate cell debris. Lipoprotein-OspA is then eluted and filtered to remove any residual *E. coli.* Secondly, diafiltration is used to eliminate the SB12 detergent and to concentrate the product. Thirdly, the retentate is applied on an ion exchange Q Sepharose column in the presence of Triton X-100. Lipoprotein-OspA is not bound to the column and the flow through is injected onto a SP Sepharose column. The column is washed and lipoprotein-OspA is eluted to be injected on a Sephacryl S300 HR gel permeation column to exchange buffer and eliminate Triton X-100. The purified lipoprotein-OspA is diluted to a standard concentration and sterilized by filtration (0.2um). The following steps are described in more detail below. All purification steps after Streamline SP take place in a cold room at +2 to +8°C.

**Streamline SP:**

Following fermentation, the diluted cell homogenate was applied to an expanded bed Streamline SP (Pharmacia, Sweden) column equilibrated in TSTA buffer (25mM NaCl, 25mM acetate, pH 4.8, 3% SB12). The column was washed with TSTA buffer to remove all unbound material. Next the column was packed in the same buffer by inverting the flow. Elution occurred with the TSTC buffer (25mM citrate, pH 6.0, 0.25% SB12). The product was pooled on basis of the UV absorption chromatogram at 280 nm.

The pH of the Streamline SP eluate was adjusted to 7.9 ± 0.3 with a diethanolamine solution and filtered on a 0.2 um membrane (Sartobran 0.45 um + 0.2 um, 4500 cm$^2$) to eliminate potential residual *E. coli.* Following filtration the Streamline SP eluate can be stored overnight at +2 to +8°C before diafiltration.

**Diafiltration:**

Diafiltration uses a plate and frame device. The Filtron Omega membrane (low binding polyethersulfone) with a 30 Kd cut-off was used.

The first step concentrates the lipoprotein-OspA containing eluate approximately 10 fold, from approximately 240 L to approximately 24 L followed by a diafiltration against minimal 10 volumes (approximately 240 L) of diethanolamine buffer (25mM DEA pH 8.6). Triton X-100 (to maintain solubility of the lipoprotein-OspA) was added to the retentate up to a concentration of 1%.

The retentate can be left standing overnight at +2 to +8°C before running the Q Sepharose column.

**Anion Exchange - Q Sepharose:**

A Q Sepharose FF (Pharmacia, Sweden) column was equilibrated with a diethanolamine buffer (TQA containing 25mM DEA pH 8.6, 0.1% Triton X-100). The diafiltration retentate was loaded on the column and the flow through was collected, including a wash step with TQA buffer, until the UV signal at 280 nm returns to baseline. The pH of the flow through is lowered to pH 4.8 with acetic acid.

**Cation Exchange - SP Sepharose:**

A SP Sepharose FF (Pharmacia, Sweden) column was equilibrated in TSPA buffer (25mM acetate, pH 4.8, 0.1% Triton X-100) and then the flow through and wash fraction was applied. The column was washed with 2 column volumes of the same TSPA buffer. Lipoprotein-OspA is eluted with 25mM citric acid pH 5.7, 0.1% Triton X-100 (TSPC buffer).

**Gel Filtration - Sephacryl S300 HR:**

The SP Sepharose eluate was injected (max. 9.5 L) onto a Sephacryl S300 HR (Pharmacia, Sweden) gel permeation column. The column was run in a 10mM $PO_4$, 150 mM NaCl pH 6.8 buffer (THR). Samples of the eluate fractions were taken and stored at + 2 to +8°C for in-process control analysis or at -70°C. The lipoprotein-OspA peak was pooled on the basis of the HPLC analysis. For HPLC, a Zorbax GF450 column in 200 mL phosphate buffer, pH 7.6 was used. The flowrate was 1 mL/min and sample volume was 10 ul. Optical density was measured at 210 nm. Pooling was started when surface of shoulder peak ≤ 1.2 % of lipoprotein-OspA peak; pooling was ended when concentration of lipoprotein-OspA < 400 ug/mL.

**Sterile Filtration:**

The Sephacryl S300 HR fraction pool containing the lipoprotein-OspA peak was diluted to a concentration of 1mg protein/mL (target value) with the same THR buffer and immediately sterile filtered.

The diluted purified lipoprotein-OspA was sterilized by filtration on a 0.2 um membrane. After filtration samples are withdrawn and stored at + 2 to +8°C for in-process and QC analysis.

The sterile purified antigen was stored at +2 to +8°C until formulation.

Purification of lipoprotein OspA from *Borrelia garinii* strain ZQ1 or *Borrelia afzelli* strain ACA-1 can also be performed using the following process, however the yields are lower than for ZS7 OspA, presumably due to the higher pK's of the ZQ1 and ACA-1 OspA proteins. Elimination or modification of the acid step in the beginning of the process improves the yield approximately 3 fold.

Table 1 outlines the purification of lipoprotein OspA ZS7, expressed in *E. coli*.

Table 1:

| Purification of OspA: | |
| --- | --- |
| Different Steps | Buffer Composition |
| Cell disruption Dilution/acidification | TBDI |
| Streamline ™ SP (cation exchange) | TSTC |
| Sterile filtration Diafiltration | TQA |
| Q-Sepharose FF (anion exchange) | TQA |
| SP-Sepharose FF (cation exchange) | TSPC |
| Sephacryl S 300 HR (gel permeation) | THR |
| Sterile Filtration | |
| TBDI: 25 mM acetic acid, pH 4.8, 4mM SB12 TSTC: 25 mM citric acid, pH 6.0, 0.25% SB 12 TQA: 25mM DEA, pH 8.6 TSPC: 25 mM citric acid, pH 5.7, 0.1% triton X THR: $Na_2HPO_4/K_2HPO_4$ 10mM, NaCl 150 mM | |

**Example 2: Purification of Lipoprotein OspA - Scheme II:**

**Fermentation:**

As in example 1, full-length OspA was expressed *E. coli* (strain AR58), which had been transformed with pOA15 carrying the coding sequence for *Borrelia garinii* strain ZQ1 lipoprotein OspA (see WO93/04175) under the control of the heat inducible lambda pL promoter. Induction was at 39.5°C +/- 2.0°C for approximately 23 hours. The fermentation broth was clarified by a tubular bowl (Sharpless) centrifuge. The pellet is recovered from the bowl and frozen at -70°C. The pellet may be stored at -70°C for up to 12 months prior to further processing.

Cells are thawed and resuspended in a cell disruption buffer containing SB12 detergent (10mBisTris, pH 6.5, 3% SB12) to an equivalent of $OD_{650} = 60$ (theoretical value calculated from the $OD_{650}$ obtained at the end of fermentation-equivalent to 30 g dry weight /liter) for 2 hours with stirring at room temperature (20-25°C).

Following fermentation and cell lysis, the impure solution was centrifuged (17000 x g, 30min) and filtered (1.2um, followed by 0.45um and 0.2um) to eliminate potential residual *E. coli*.

**Purification:**

Purification is achieved by three chromatographic steps. Preferably, an impure solution containing lipoprotein-OspA is centrifuged and filtered remove any residual *E. coli.* Then the impure solution is applied on an anion exchange (Q Sepharose FF or XL) column in the presence of Triton X-100. Lipoprotein-OspA is not bound to the column and the flow through is injected onto a cation (SP Sepharose or CM Sepharose) column. The column is washed and lipoprotein-OspA is eluted to be injected on a Sephacryl S300 HR gel permeation column to exchange buffer and eliminate Triton X-100. The purified lipoprotein-OspA is diluted to a standard concentration and sterilized by filtration (0.2um). The following steps are described in more detail below.

All purification steps take place in a cold room at +2 to +8°C.

**Anion Exchange - Q Sepharose:**

A Q Sepharose FF (Pharmacia, Sweden) column was equilibrated with a diethanolamine buffer (TQA containing 25mM DEA pH 8.8, 0.1% Triton X-100). The impure solution was loaded on the column and the flow through was collected, including a wash step with TQA buffer, until the UV signal returns to baseline. The pH of the flow through is lowered to pH 4.8 with acetic acid.

**Cation Exchange - SP Sepharose:**

A SP Sepharose FF (Pharmacia, Sweden) column was equilibrated in TSPA buffer (25mM acetate, pH 4.8, 0.1% Triton X-100) and then the flow through and wash fraction was applied. The column was washed with 2 column volumes of the same TSPA buffer. Lipoprotein-OspA is eluted with 25mM acetate, 0.2M NaCl, pH 4.8, 0.1% Triton X-100.

**Gel Filtration - Sephacryl S300 HR:**

The SP Sepharose eluate was injected (max. 9.5 L) onto a Sephacryl S300 HR (Pharmacia, Sweden) gel permeation column. The column was run in a 10mM $PO_4$, 150 mM NaCl pH 6.8 buffer (THR). Samples of the eluate fractions were taken and stored at + 2 to +8°C for in-process control analysis or at -70°C. The lipoprotein-OspA peak was pooled on the basis of the HPLC analysis. For HPLC, a Zorbax GF450 column in 200 mL phosphate buffer, pH 7.6 was used. The flowrate was 1 mL/min and sample volume was 10 ul. Optical density was measured at 210 nm. Pooling was started when surface of shoulder peak $\leq$ 1.2 % of lipoprotein-OspA peak; pooling was ended when concentration of lipoprotein-OspA < 400 ug/mL.

**Sterile Filtration:**

The Sephacryl S300 HR fraction pool containing the lipoprotein-OspA peak was diluted to a concentration of 1mg protein/mL (target value) with the same THR buffer and immediately sterile filtered.

The diluted purified lipoprotein-OspA was sterilized by filtration on a 0.2 um membrane. After filtration samples are withdrawn and stored at + 2 to +8°C for in-process and QC analysis.

The sterile purified antigen was stored at +2 to +8°C until formulation.

**Example 3: Purification of Lipoprotein OspA - Scheme IIA:**

**Fermentation:**

As in example 1, full-length OspA was expressed *E. coli (strain* AR58), which had been transformed with pOA15 carrying the coding sequence for *Borrelia afzelli* strain ACA-1 lipoprotein OspA (see, US Patent 5,777,095) under the control of the heat inducible lambda pL promoter. Induction was at 39.5°C +/- 2.0°C for approximately 23 hours.

The fermentation broth was clarified by a tubular bowl (Sharpless) centrifuge. The pellet is recovered from the bowl and frozen at -70°C. The pellet may be stored at -70°C for up to 12 months prior to further processing.

Cells are thawed and resuspended in a cell disruption buffer containing SB12 detergent (10mMBisTris, pH 6.5, 3% SB12) to an equivalent of $OD_{650}$ = 60 (theoretical value calculated from the $OD_{650}$ obtained at the end of fermentation-equivalent to 30 g dry weight /liter) for 2 hours with stirring at room temperature (20-25°C).

Following fermentation and cell lysis, the impure solution was centrifuged (17000 x g, 30min) and filtered (1.2um, followed by 0.45um and 0.2um) to eliminate potential residual *E. coli.*

**Purification:**

As in Example 2, purification is achieved by three chromatographic steps with some minor modifications.

**Anion Exchange - Q Sepharose:**

A Q Sepharose XL (Pharmacia, Sweden) column was equilibrated with a diethanolamine buffer (TQA containing 25mM DEA pH 9.1, 0.1% Triton X-100). The impure solution was loaded on the column and the flow through was collected, including a wash step with TQA buffer, until the UV signal at 280nm returns to baseline. The pH of the flow through is lowered to pH 4.8 with acetic acid.

**Cation Exchange - CM Sepharose:**

A CM Sepharose FF (Pharmacia, Sweden) column was equilibrated in TSPA buffer (25mM acetate, pH 4.8, 0.1% Triton X-100) and then the flow through and wash fraction was applied. The column was washed with 2 column volumes of the same TSPA buffer. Lipoprotein-OspA is eluted with 50mM citrate pH 6.2, 0.1% Triton X-100.

**Gel Filtration - Sephacryl S300 HR:**

The CM Sepharose eluate was injected (max. 9.5 L) onto a Sephacryl S300 HR (Pharmacia, Sweden) gel permeation column. The column was run in a 10mM $PO_4$, 150 mM NaCl pH 6.8 buffer (THR). Samples of the eluate fractions were taken and stored at + 2 to +8°C for in-process control analysis or at -70°C. The lipoprotein-OspA peak was pooled on the basis of the HPLC analysis. For HPLC, a Zorbax GF450 column in 200 mL phosphate buffer, pH 7.6 was used. The flowrate was 1 mL/min and sample volume was 10 ul. Optical density was measured at 210 nm. Pooling was started when surface of shoulder peak ≤ 1.2 % of lipoprotein-OspA peak; pooling was ended when concentration of lipoprotein-OspA <400 ug/mL.

**Sterile Filtration:**

The Sephacryl S300 HR fraction pool containing the lipoprotein-OspA peak was diluted to a concentration of 1mg protein/mL (target value) with the same THR buffer and immediately sterile filtered.
The diluted purified lipoprotein-OspA was sterilized by filtration on a 0.2 um membrane. After filtration samples are withdrawn and stored at + 2 to +8°C for in-process and QC analysis.
The sterile purified antigen was stored at +2 to +8°C until formulation.
Lipoprotein OspA from *Borrelia burgdorferi* sensu stricto strain ZS7 has also been purified using Scheme II

**Example 4: Analysis of Contaminants:**

**4.1. E. coli contaminants via ELISA and Western Blot:**

Polyclonal antibodies were prepared and used as follows.

**4.1.1. Anti-<u>E. coli</u> polyclonal antibody production:**

A crude extract of heat-induced wild type *E. coli* parental strain AR58 was used to produce the antibody. This strain does not contain a plasmid coding for lipo-OspA. Three New Zealand white rabbits were injected subcutaneously with 100 μg of the crude extract in complete Freund's adjuvant. Animals were boosted 3 times by injecting 100 μg of antigen in incomplete Freund's adjuvant (IFA) on day 21, 100 μg in PBS (phosphate buffer saline) on day 42 and 100 μg in IFA on day 109. Individual immune sera were collected on day 123 (14 days after the last boost) and tested by Western blot (WB). They showed a recognition pattern of about 20 distinct bands ranging from 200 to 20 kDa. As the recognition pattern showed slight differences between the three immune sera, the 3 sera were pooled in order to optimize the recognition profile. This pool was used as such in Western Blot analysis. Part of the anti-*E. coli* specific antibodies were purified by affinity chromatography on protein A sepharose column (p-Rb-IgG) and characterized by SDS-PAGE to confirm the absence of contaminating rabbit serum albumin. A fraction of the purified antibody was biotinylated according to a standard procedure (Amersham Kit) (biotinylated p-Rb-IgG) and used at a 1/200 working dilution in the ELISA assay.

### 4.1.2. Characterization of the anti-<u>E coli</u> polyclonal antibody:

Further characterization of the purified polyclonal antibody (p-Rb-IgG) was performed by WB and SDS-PAGE analysis. To test for potential cross reactions, the polyclonal antibody specific for the *E. coli* protein extract and monoclonal antibodies against lipo-OspA (LA-2 and LA-31 (see US Patent 5,780,030), were tested against the *E. coli* protein extract (EPE), the vaccine antigen (lipo-OspA) and a mixture of both lipo-OspA and EPE.

The polyclonal antibodies were specific for *E. coli* proteins and showed a similar recognition profile when EPE was mixed or not with lipo-OspA, indicating that lipo-OspA does interfere with the detection of *E. coli* proteins. The *E. coli* proteins are therefore specifically detected by the polyclonal rabbit *E. coli* proteins.

### 4.1.3. Western blot analysis:

The host cell derived protein level was qualitatively assessed by Western blot analysis. The proteinic samples were reduced in the presence of β-mercaptoethanol, denatured in SDS sample buffer and loaded at 20 μg/lane on a 12.5% SDS-PAGE gel. A series of dilutions of the EPE (20, 10, 5 and 1 μg per lane) was loaded as well. Following electrophoresis, the proteins were transferred to a nitrocellulose membrane using a transfer buffer (Tris 0.025 M, pH 8.3, glycine 0.192 M containing 20% methanol and 0.1% SDS). The membrane was then blocked with the incubation buffer (PBS containing 0.1% tween 20) and incubated during 1 hour in the presence of the polyclonal anti-*E. coli* antiserum diluted 250-fold in the incubation buffer. All steps were performed at room temperature on a rocking platform. After incubation with the antibody, the membrane was washed three times in the incubation buffer (5 minutes each wash) and then incubated during 1 hour with an anti-rabbit Ig biotinylated species-specific whole antibody from donkey, diluted 1/500 in the incubation buffer. After the washings and addition of a streptavidin-biotinylated horseradish peroxidase reagent (1/1000 dilution in the incubation buffer), the antigen-antibody complex was revealed by addition of 4-chloro-1-naphtol/methanol/$H_2O_2$ solution and the reaction stopped by the addition of water.

### 4.1.4. ELISA assay:

To quantitatively evaluate the host cell derived protein level of samples, an enzyme-linked immunosorbent assay (ELISA) was developed.

Microtiter plates are coated for 2 hours at 37°C with purified rabbit anti-E. *coli* IgG fraction (p-Rb-IgG) dissolved in bicarbonate buffer (pH = 9.6). Between different steps, plates are washed with PBS containing 0.1% tween 20. Plates are blocked with 1% BSA (Bovine serum albumin) in PBS. Test samples (or reference) are serially diluted (in PBS containing 0.2% BSA) and incubated 90 min at 37°C. Plates are then incubated with the diluted biotinylated p-Rb-IgG for 1 hour at 37°C. Streptavidin-biotinylated peroxidase reagent (in 0.2 % BSA-PBS) is added to each well and plates are incubated for 30 min at room temperature. Substrate (TMB = 3,3',5,5'-tetramethylbenzidine) solution is then added to reveal the antibody-antigen complex and the reaction stopped by addition of $H_2SO_4$. Absorbance is determined at 405 nm.

*E. coli* concentration in samples was calculated relative to a reference curve of EPE (2-fold dilutions from 20 μg per ml) established using the four-parameter logistic equation (Karpinski KF et al., 1987, J. Immunology Methods 103:189). A 20% -80% calculation program (SOFTmaxPRO) is applied to the lower and upper asymptotic OD values. All samples have been two-fold diluted starting from 200 μg of protein per ml (by Lowry assay). The average of the concentrations obtained for the different dilutions is calculated, taking into consideration all OD values belonging to the linear part of the reference curve (20% - 80% cut-off). Results have been confirmed using a linear regression method.

The detection limit of the ELISA assay (mean OD 405 of blank [PBS containing 0.2% BSA] + 3 standard deviations) was shown to be 50 ng of the *E. coli* protein extract per ml. Given that samples have been tested at a maximum concentration of 200 μg protein per ml (first dilution), the lowest level of *E. coli* proteins that can be detected is 0.025 % of the total protein content.

### 4.1.5 Conclusion:

Based on the experiments described above, using both Western blot analysis and an ELISA assay, it is demonstrated that clearance of *E. coli* protein contaminants from fermentation product under Example 1 is almost complete after the SPC (flow through of SP-Sepharose FF (cation exchange) column) step. The residual *E. coli* contamination is estimated to be less than 0.025 % by ELISA. The purification process is reproducible as has been demonstrated on the 20 L batches and at the 75L scale. Similar results are produced under Examples 2 and 3.

**4.2. DNA quantitation:**

**4.2.1:**

The method used for DNA quantitation in the different samples was the Threshold™ system (Molecular Devices Corp.). This system includes a semiautomated workstation, a reader, a personal computer, software, all reagents and disposables required for DNA determinations and a procedure to conduct the test.

The detection of DNA is based on the recognition of single-stranded DNA, obtained after proteinase K treatment and heat denaturation, by two DNA binding proteins in a sandwich assay with streptavidin. One of the proteins, single-stranded DNA-binding protein (SSB) from *E. coli* is linked to biotin for specific capture of the DNA complex onto a membrane, the other is an anti-DNA monoclonal antibody conjugated to urease for signal generation.

Both proteins have a high affinity for single-stranded DNA but low sequence specificity and form with DNA and streptavidin a streptavidin-biotin-SSB-DNA-antibody-urease complex.

This complex is captured on a biotinylated membrane through filtration under vacuum. The unbound reagents are eliminated by washing.

Since the amount of urease captured is proportional to the amount of DNA, urease activity can be used to quantitate the DNA. The rate of conversion of urea to ammonia and carbon dioxide is determined by a pH sensitive semiconductor.

The amount of DNA in the samples is determined against a standard curve from 2 pg to 200 pg of calf thymus DNA calibrator. The lowest amount that can be quantified therefore is 2 pg.

**4.2.2 Sample treatment:**

Each volume of test sample was treated by proteinase K (0.1 mg/ml) and SDS (0.1%) at 55°C for 16 hours in a final volume of 500 μl before denaturation at 100°C for 20 minutes and labeling reaction according to the manufacturer's protocol.

In order to validate the pretreatment and monitor any inhibitory effects associated with the sample, a spike recovery test was performed on each sample by adding 50 pg of exogenous DNA (according to manufacturer's protocol) and by demonstrating a quantitative recovery as compared to a control sample containing 50 pg of calf thymus DNA in buffer.

The recovery of DNA in the spiked sample is calculated using the following formula:

$$\% \text{ recovery} = \frac{\text{(signal spiked sample - signal of sample)}}{\text{(signal spiked buffer - signal of buffer)}} \times 100.$$

For a test to be valid, the recovery of DNA in the spiked sample must be between 80 and 120%.

**4.2.3. Results:**

The Q-Sepharose column is the most efficient process step in the removal of DNA.

In the purified OspA samples from Example 1, the quantity of residual DNA in 100 μl of the undiluted samples is close to the detection limit of the system (2 pg). Taking into account the protein concentration of the purified bulk (approximately 1 mg/ml), this corresponds to 2 pg per 100 μg of antigen. The DNA concentration in a vaccine dose of 30 μg lipo-OspA will therefore also correspond to less than 2 pg. Similar results are found with samples from Examples 2 and 3.

**4.3 Evaluation of clearance of SB 12 by the purification process of lipo-OspA: Residual level of SB12 in the purified protein:**

**4.3.1. Introduction:**

The extraction of lipo-OspA from *E. coli* cells uses a buffer containing the detergent *N*-dodecyl-*N,N'*-dimethyl-3-ammonio-1-propanesulfonate (lauryl-sulfobetain or SB12). Presence of 3% (w/v) of this detergent in the cell extract keeps the lipo-OspA soluble for the subsequent purification steps.

Experiments were performed to assess the extent of clearance of SB12 from the product by the different purification steps and to quantify residual SB12 at the purified bulk stage.

**4.3.2. Analytical method for the assay of SB12:**

**4.3.2.1. Principle of the method:**

SB12 is separated from buffer components (phosphate salts) by reversed phase HPLC on a C18 column and the column eluate injected in an ESMS (Electrospray Mass Spectrometer). The substance is detected by SIM (single ion monitoring) at m/z 336, the molecular ion of SB12.

**4.3.2.2. Experimental conditions:**

The HPLC system consisted of an Applied Biosystems 140 solvent delivery system, a Vydac C18 column (250 x 2.1 mm), the ESMS and an Applied Biosystems 759 A Absorbance detector set at 214 nm. The column eluate is split in a ratio 90/10: 10% to the ESMS, 90% to the UV-detector. A by-pass system is installed after the column, so that the injection peak containing the salts is eliminated. The use of such a system increases the reproducibility of the method and avoids clogging of the source. Mobile phase (flow rate 200 μl per min) consisted of methanol:water 74:10 containing 0.1% of trifluoroacetic acid. Injection volume was 20 μl.

ESMS (VG Platform II, Micromass) was performed in the positive ion mode (ES+) and the molecular ion of SB12 (m/z 336) was monitored as a single ion to obtain the highest possible sensitivity. Tuning parameters of the ESMS were as follows:

*Source:*   capillary 3.5 kV, HV lens 0.5 kV, cone 45 V, source temperature 60-120°C
*MS:*   ion energy 1 V, LM resolution 10, HM resolution 10, multiplier 650 V
*SIR:*   mean mass 336, dwell time 0.2 sec, interchannel delay 0.02 sec.
   For calibration of the mass scale, the clusters of NaI are used.

**4.3.2.3. Standard curve:**

A stock solution containing 10 ppm of SB12 is diluted so as to obtain solutions at 5.0, 2.5, 1.0, 0.50, 0.25, 0.100, 0.050, 0.025 and 0.01 ppm. The data of a standard curve are given in Table 2.

Table 2:

| Data obtained for a calibration curve of SB12 by ESMS: | | | |
|---|---|---|---|
| Concentration (Ppm) | Mean area (n = 3) | SD | RSD (%) |
| 10.0 | 2384603.7 | 236593.3 | 9.9 |
| 5.0 | 1186395.3 | 23815.5 | 2.0 |
| 2.5 | 509954.0 | 11285.7 | 2.2 |
| 1.0 | 278388(n=1) | n = 1 | n = 1 |
| 0.500 | 110229.0 | 8318.0 | 7.5 |
| 0.250 | 62111.7 | 3797.4 | 6.1 |
| 0.100 | 32615.3 | 2518.6 | 7.7 |
| 0.050 | 12902.7 | 1627.6 | 12.6 |
| 0.025 | 5915.7 | 1066.5 | 18.0 |
| 0.01 | 1736.3 | 477.5 | 27.5 |

**4.3.2.4. Sample preparation:**

When possible, samples were injected as such, without dilution, to obtain the highest possible sensitivity. In the first steps of the purification process, where higher concentrations of SB12 are present, an appropriate dilution (1000 x, 100 x or 10 x) was made in order to obtain a response within the range of the calibration curve.

### 4.3.3. Assessment of removal of SB12 by the purification process:

Different samples of intermediate steps in the purification process of different batches have been analyzed for their SB12 content. Table 3 shows the results obtained on the 75 L consistency batches at the different stages of the purification process.

Table 3:

| SB12 concentration (ppm) at different purification steps: 75 L batches: | | | |
|---|---|---|---|
| Purification intermediate | Batch OPA113 | Batch OPA114 | Batch OPA115 |
| BZ5S | 28102 | 29683 | 35759 |
| STC | 3153 | 2680 | 4120 |
| RD | 100 | 60 | 90 |
| QFT | 29 | 21 | 31 |
| SPC | < 0.025 | 0.07 | 0.06 |

As can be seen, the concentration of SB12 found in the same purification intermediates is consistent for 75 L and 20 L batches. The level of SB12 present after SP Sepharose is below 100 ppb (0.100 ppm).
Clearance for all steps is more than 90% except for the Q Sepharose step, which does not remove SB12. The lower concentration after this step is only due to a higher process volume (dilution effect).
Figure 1 graphically represents the reduction in SB12 concentration by the different purification steps. The elimination is consistent for all the 75 L and 20 L lots studied.

### 4.3.4 Conclusion:

Using a very sensitive and accurate method for the detection of SB12, it has been demonstrated that the residual level of SB12 in the purified lipo-OspA bulk is below 10 ppb. Analysis of different intermediates of the purification step, at both the 20 L and 75 L scales, shows that the purification process consistently removes SB12 from the initial extract.

### 4.4 Triton X-100 Content by Colorimetry:

### 4.4.1. Introduction:

This section describes the results of experiments performed to validate the assay for Triton content in lipo-OspA purified bulk.
The colorimetric method is described below:

### 4.4.2 Method of Analysis:

The method used is based on the publication by Huddleston, R.L. and Allred, R.C., (J. Am. Oil Chemist Soc., 1965, 42, 983).
The assay is based on a procedure also used for the determination of non-ionic surfactants. The surfactant is complexed with cobalt thiocyanate. The colored complex is extracted with ethylene dichloride and this absorbance is measured at 320 nm.

### Reagents and standard solutions:

### 1. Reagents:

Ethylene dichloride, Cobalt nitrate $6H_2O$, Ammonium thiocyanate, 98% Formic acid.

**2. Cobalt thiocyanate reagent:**

Dissolve 12.7 g ammonium thiocyanate and 2.0 g of cobalt nitrate hexahydrate in 100 ml of purified water and mix well.

**3. Standard solution:**

Accurately weigh 100 mg of Triton X100 into a 100 ml volumetric flask. Dissolve and dilute to volume with ethylene dichloride.

**Procedure:**

**A. Calibration:**

Prepare a range of standard solutions containing 0, 2.5, 5, 10, 15, 20, 25, 30 and 40 μg/ml of Triton X 100 in ethylene dichloride. To 1 ml of each standard solution, add 100 μl of formic acid and 1 ml of cobaltothiocyanate reagent. Mix well for about 20 minutes.
Centrifuge for approximately 15 minutes at about 5000 rpm, withdraw and discard the upper (aqueous) layer. Measure the absorbance of the lower (ethylene dichloride) layer at 320 nm in microcuvettes.
Plot the concentration (μg/ml of Triton X100) against the absorbance (calibration curve).

**B. Sample treatment:**

Transfer, in duplicate, 1 ml of sample to the tubes and evaporate to dryness in a Savant Speed Vac concentrator.
To the residue, add 100 μl of formic acid and 1 ml of ethylene dichloride. Take the residue in suspension by sonication.
Into the first tube, add 1 ml of cobaltothiocyanate reagent (test tube). Into the second tube, add 1 ml of purified water (blank tube). Mix well for about 20 minutes.
Centrifuge for approximately 15 minutes at about 5000 rpm, withdraw and discard the upper (aqueous) layer. Measure the absorbance of the lower (ethylene dichloride) layer at 320 nm in microcuvettes.
Calculate the difference in O.D. between the value obtained with the test tube and the O.D. value of the blank. Determine the triton content of the sample from the calibration curve.

**Limit of detection:**

2 μg per ml.

**4.4.3. Validation of Method of Analysis:**

**4.4.3.1. Limit of detection (LOD):**

Calculation based on the calibration curve
Calibration curves have been determined on four different days (3 to 5 replicates of dilutions per day). The mean and standard deviation (SD) of the y-intercepts of the regression lines obtained on each day were determined and the mean + 3 SD was calculated. Based on the average value of each day and the average calibration curve for that day, the LOD was calculated. The results for the four days are reported in Table 4. The average of the results obtained on the four days is 1.8 ug/ml.

**4.4.3.2 Calculation based on the standard deviation and the slope:**

$$LOD = 3.3 \, SD / S$$

SD = the standard deviation of the y-intercepts
S = slope of the average calibration curve

The results obtained on the four days are given in Table 4. As can be seen the average of the four values is 1.9 ug/ml which is very close to the value obtained under 4.4.3.1.
Based on these experiments, it can be concluded that the LOD is 2 ug/ml.

**4.4.3.3 Limit of quantification (LOQ):**

The limit of quantification is calculated using the results obtained in the experiments described under 4.4.3.1.

$$LOQ = 10 \, SD / S^{1}$$

SD = the standard deviation of the y-intercepts
S = slope of the average calibration curve

The individual results are summarized in Table 4. It can be concluded that the LOQ is 6 ug/ml.

### Table 4: Limit of detection and limit of quantification:

| | Limit of detection (ug/ml) | | Limit of quantification (ug/ml) |
|---|---|---|---|
| Day | Mean + 3 SD (y-intercept) | LOD = 3.3 SD/S | LOQ = 10 SD/S |
| 1 | 2 | 2.3 | 6.8 |
| 2 | 3 | 3.4 | 10.3 |
| 3 | 1.3 | 1.3 | 4 |
| 4 | 1 | 0.7 | 2.2 |
| MEAN | 1.8 | 1.9 | 5.8 |

**4.4.3.4 Linearity and range:**

To assess the linearity of the colorimetric method and the concentration range over which Triton content can be determined in an accurate and precise way, three calibration curves were established over the range 0 to 40 ug of Triton per ml. The data were analyzed by linear regression.
It can be concluded that within the concentration range tested (0 - 40 ug/ml), the colorimetry is linear, accurate and precise. Given the LOQ of 6 ug per ml, the range in which precise and accurate results can be obtained is 6 to 40 ug/ml. Samples with a Triton concentration above 40 ug/ml should be retested after dilution.

**4.4.3.5 Specificity:**

As absolutely "pure" lipo-OspA, containing no Triton, is not available, it is not possible to verify the specificity of the assay, by checking whether substances present in the product would interfere with the assay. However, as mentioned earlier, any interference of ingredients present in the bulk and absorbing at the same wavelength (320 nm), is accounted for by subtracting the blank (sample without cobalt reagent) in each sample analysis.
The good recoveries obtained after spiking at two different concentrations also indicate that there is no major interference from components other than Triton.

### 4.4.3.6 Conclusion:

Based on the data presented above, it can be concluded that the test method for the assay of Triton in lipo-OspA bulk is suitable for its intended use : assay of residual triton in lipo-OspA bulk with a specification limit of maximum 10 ug/ml.

The colorimetric method gives precise and accurate results for a range between 6 and 40 ug per ml. It should be noted that the final result of the assay is the difference between two experimental values and therefore can be reported as a value below 6 ug per mL. The limit of detection is 2 ug/mL. Any interference of sample ingredients absorbing at 320 nm is eliminated by using a blank. The color of the Triton-Cobalt complex is stable for at least 2 hours.

### Example 5. Characterization of the Lipoprotein OspA:

### Endotoxin: Routine Testing with Chromogenic LAL Method:

The purified OspA antigen is routinely tested for endotoxin content by the chromogenic LAL (limulus amebocyte lysate) test with a specification of $\leq 5$ EU per 30 $\mu$g of protein. The test is carried out and validated in conformity with the FDA Interim Guidance for Human and Veterinary Drug Products and Biologicals on Kinetic LAL Techniques, as described in the Guideline on Validation of the Limulus Amebocyte Lysate Test as an End-Product Endotoxin Test for Human and Animal Parenteral Drugs, Biological Products, and Medical Devices (December 1987).

### Triton X-100: Routine Testing with Colorimetric Method:

As Triton X-100 is added at an intermediate step of the purification process (before the Q Sepharose column), its content in the purified bulk antigen is routinely quantified by a colorimetric method. The amount of Triton X-100 in a 1 mg/ml solution of lipoprotein OspA is no more than 10 $\mu$g per ml (10 ppm or less). Based on a 30ug dose of OspA, the residual quantities in the finished product would be lower than 300 ng/dose (based on the acceptance criterion of less than 10 $\mu$g/ml).

A literature search for Triton toxicity studies yielded no intramuscular toxicity studies. The mouse $LD_{50}$ for Triton X-100 after intravenous administration was reported to be 1200 mg/kg.

### *E. coli* Contaminants: Clearance Study Using an ELISA and Western Blot Method:

To assess the efficacy of the purification process in removing *E. coli* contaminants, a clearance study was performed using two analytical techniques. Both Western blot and ELISA showed that the purification process consistently eliminates contaminant proteins and that at the end of the purification process, very low amounts of *E. coli* contaminants are left. The ELISA assay allowed an estimate of the residual amount of *E. coli* proteins (<0.025 %).

### DNA: Clearance Study Using the Threshold Method:

As for *E. coli* contaminants, a study was undertaken to assess the clearance of DNA by the different purification steps and to quantify residual DNA in the purified bulk antigen. It can be concluded that DNA is efficiently and consistently removed by the purification process (a reduction of about 5 log is obtained). Residual DNA is very low, resulting in less than 2 pg of DNA per 30ug (of OspA) vaccine dose. This is well below the level recommended by a WHO consultative group, i.e. 100 pg of cellular DNA per dose (WHO Technical Report Series, 747, 1987).

### SB12: Clearance Study using an HPLC-ESMS Method:

The results of the clearance study demonstrate that the purification process consistently removes SB12 from the harvest fluid (clearance factor about 7 log), resulting in less than 10 ppb of SB12 in the purified product. This amount is far below that described (37.5 $\mu$g/ml) in literature references as not having toxicological effects. [Speijers et al., Vaccine, 7:364, 1989; Speijers et al., Vaccine, 6:419, 1988; Ernst and Arditti, Toxicology, *15:233,* 1980].

### Example 6. Purity and Stability of Lipoprotein OspA:

### SDS-PAGE:

Several lots (2x75L, 3x20L) of ZS7 OspA were analyzed on SDS-PAGE. Each lot analyzed showed a major band with an apparent molecular weight of 31 kD. Both purity and homogeneity of OspA are demonstrated by detection of a single major band. Treatment of samples from 75L lots were incubated at 37°C for one week without effecting the SDS-PAGE pattern, indicating stability of the protein. Similar results were found for OspA from strains ZQ1 and ACA-1, however the OspA proteins from both strains had an apparent molecular weight of 30kD. Also observed for both proteins was a minor dimer band at 60kD.

**Western Blotting:**

Several lots (2x75L, 2x20L) of ZS7 OspA were analyzed by Western Blotting with monoclonal antibody LA-2, an OspA specific antibody. Each lot analyzed showed a major band with an apparent molecular weight of 31 kD. Both purity and homogeneity of OspA are demonstrated by the presence of a single major band. Treatment of samples from 75L lots were incubated at 37°C for one week without effecting the Western Blot pattern.

**N-Terminal Sequence Analysis:**

N-terminal sequence analysis was performed on two 20L and two 75L lots of OspA from strain ZS7 and 3 lots of OspA from strain ACA-1, stored at 4°C and after a 7 day incubation at 37°C. The N-terminal sequence of the non-modified lipoprotein OspA (i.e., prolipoprotein with signal sequence) was very weakly detected. The sequence of non-lipidated OspA was also identified but at a very low level. The very low levels of non-modified and non-lipidated (incompletely modified) OspA (i.e., less than 1 % of the total amount) as well as the absence of breakdown products and of other protein contaminants shown by this technique confirm the high degree of purity of OspA. The concurrent data among all lots further supports consistency and homogeneity of the purification process.

**Laser Light Scattering Analysis:**

The laser light scattering detector measures the light intensities scattered at 15 angles by a macromolecular solution. From these intensities, the size and shape of the macromolecule in solution can be determined. The detector is coupled to a HPLC size exclusion column from which the lipoprotein is eluted. An interferometric refractometer placed on-line allows the determination of the quantity of the lipoprotein eluted. The mean molecular weight ($M_w$) is defined as the sum of the weights of all species multiplied by their respective molecular weight and divided by the sum of the weights of all species.

A calculated mean molecular weight around 500kD (ranging from 411 to 581kD) was found for the two 75L lots tested before and after 7 days at 37°C for OspA from strain ZS7. For OspA from strain ACA-1, the mean molecular weight is around 450kD based on three lots. There was no significant difference between time 0 and 7 days at 37°C.

Further studies were conduced on three batches of lipoprotein OspA from strain ZS7 after 12 months storage at 4°C. After 12 months, the major OspA population had a molecular weight between 450 and 550kD, showing stability of the OspA micellular structure.

**Mass Spectrometry:**

The molecular mass from three batches of lipoprotein OspA (from strain ZS7) stored at 4°C for 12 months was determined by electrospray ionization (Platform II instrument). The mean molecular mass of the major peak was 28,523.13 +/- 0.52 Daltons, which is well in agreement with the expected value of 28,523.1 Daltons. There is also a smaller peak at 28,284.81 +/-1.32 Daltons, which corresponds with the loss of one palmitoyl lipid chain. No other masses, corresponding to other degradation products were observed after 1 year at 4°C showing the stability of OspA.

**Determination of Lipid Content:**

The lipid content from three batches of lipoprotein OspA (strain ZS7) stored at 4°C for 1 year was determined by gas chromatography after alkaline hydrolysis. The released fatty acids were assayed as FAME (Fatty Acid Methyl Ester) after acid methanolysis. A gas chromatograph HP 5890 series II, equipped with a HP 7673 automatic injector and a HP 3365 chemstation module was used for data analysis. The ratio of lipid to protein was calculated before and after storage at 4°C for 1 year. Within experimental error, 2 lipid chains are released form lipoprotein OspA by alkaline hydrolysis at time = 0 and 1 year. This is consistent with the expected 2 ester-linked lipid chains of lipoprotein OspA.

**Additional Stability Studies:**

Two clinical lots were tested at 0, 6, 12 and 24 months (at 2-8 °C) by SDS-PAGE, Western Blotting, *in vivo* potency in Balb/C mice (relative potency 90% or greater for 12 months, 80% or greater between time 0 and 24 months), etc. The conclusion was that OspA is stable for up to 2 years at 2-8 °C.

**Claims**

1. A method to purify *Borrelia* lipoprotein OspA comprising:

   (a) lysis of a host cell expressing lipoprotein OspA in the presence of a zwitterionic detergent to form an impure solution containing lipoprotein OspA;
   (b) clarifying the impure solution to remove host cell debris;
   (c) contacting the impure solution with an anion exchange resin in the presence of a nonionic detergent;
   (d) collecting flow through from said anion exchange resin and contacting said flow through with a cation exchange resin; and
   (e) eluting OspA followed by a gel filtration step,

   wherein the purification occurs without heating.

2. A method to purify *Borrelia* lipoprotein OspA comprising:

   (a) cell disruption in the presence of a zwitterionic detergent to form an impure solution containing OspA;
   (b) contacting the impure solution with a fluidized bed resin, said resin comprising a cation exchange resin;
   (c) eluting OspA from said resin and diafiltration of the eluant whereby OspA is retained;
   (d) contacting the retentate with an anion exchange resin in the presence of a nonionic detergent;
   (e) collecting flow through from said anion exchange resin and contacting said fluid flow through with a cation exchange resin; and
   (f) eluting OspA followed by a gel filtration step,

   wherein the purification occurs without heating.

3. The method of claims 1 or 2 wherein clarification of the impure solution comprises centrifugation.

4. The method of claim 1 or 2 wherein the nonionic detergent is a polyoxyethylene ether.

5. The method of claim 4 wherein the polyoxyethylene ether is Triton X-100 or Triton X-114.

6. The method of claim 4 wherein the polyoxyethylene ether is Triton X-100

7. The method of claim 1 or 2 wherein the nonionic detergent is a polyoxyethylenesorbitan ester.

8. The method of claim 7 wherein the polyoxyethylenesorbitan ester is Tween 20 to Tween 80.

9. The method of claim 1 or 2 wherein purified OspA protein at a concentration of 1 mg/ml contains less than 1000 ppm (0.1%) of nonionic detergent.

10. The method of claim 9 wherein the purified OspA protein contains less than 100 ppm of nonionic detergent.

11. The method of claim 10 wherein the purified OspA protein contains less than 10 ppm of nonionic detergent.

12. The method of claim 1 or 2 wherein the zwitterionic detergent is an N-Alkyl-N,N-dimethylammonio-1-propanesulfonate.

13. The method of claim 12 wherein the N-Alkyl-N,N-dimethylammonio-1-propanesulfonate is SB8-SB18.

14. The method of claim 12 wherein the N-Alkyl-N,N-dimethylammonio-1-propanesulfonate is SB12.

15. The method of claim 1 or 2 wherein purified OspA protein at a concentration of 1 mg/ml contains less than 1000 ppb (1 ppm) of said zwitterionic detergent.

**16.** The method of claim 15 wherein the purified OspA protein contains less than 100 ppb (0.1 ppm) of said zwitterionic detergent.

**17.** The method of claim 16 wherein the purified OspA protein contains less than 10 ppb (0.01 ppm) of said zwitterionic detergent.

**18.** The method of claim 1 or 2 which additionally comprises sterile filtration after the gel filtration.

**19.** A vaccine comprising purified lipoprotein OspA obtainable by the method of claims 1 or 2, that is detergent free.

**20.** The vaccine of claim 19 which is suitable for human clinical use.

**Patentansprüche**

**1.** Verfahren zur Reinigung von Borrelia-Lipoprotein OspA, umfassend:

(a) Lyse einer Wirtszelle, die Lipoprotein OspA exprimiert, in Gegenwart eines zwitterionischen Detergens zur Bildung einer unreinen Lösung, die Lipoprotein OspA enthält;

(b) Klären der unreinen Lösung zur Entfernung von Wirtszelltrümmern;

(c) In-Kontakt-Bringen der unreinen Lösung mit einem Anionenaustauscherharz in Gegenwart eines nichtionischen Detergens;

(d) Auffangen des Durchflusses aus dem Anionenaustauscherharz und In-Kontakt-Bringen des Durchflusses mit einem Kationenaustauscherharz; und

(e) Eluieren von OspA, gefolgt von einem Gelfiltrationsschritt,

   worin die Reinigung ohne Erwärmen erfolgt.

**2.** Verfahren zur Reinigung von Borrelia-Lipoprotein OspA, umfassend:

(a) Zellaufschluss in Gegenwart eines zwitterionischen Detergens zur Bildung einer unreinen Lösung, die OspA enthält;

(b) In-Kontakt-Bringen der unreinen Lösung mit einem Fliessbettharz, wobei das Harz ein Kationenaustauscherharz umfasst;

(c) Eluieren von OspA aus dem Harz und Diafiltration des Elutionsmittels, wobei OspA zurückgehalten wird;

(d) In-Kontakt-Bringen des Retentats mit einem Anionenaustauscherharz in Gegenwart eines nichtionischen Detergens;

(e) Auffangen des Durchflusses aus dem Anionenaustauscherharz und In-Kontakt-Bringen des flüssigen Durchflusses mit einem Kationenaustauscherharz; und

(f) Eluieren von OspA, gefolgt von einem Gelfiltrationsschritt,

   worin die Reinigung ohne Erwärmen erfolgt.

**3.** Verfahren gemäss Anspruch 1 oder 2, worin die Klärung der unreinen Lösung Zentrifugieren umfasst.

**4.** Verfahren gemäss Anspruch 1 oder 2, worin das nichtionische Detergens ein Polyoxyethylenether ist.

**5.** Verfahren gemäss Anspruch 4, worin der Polyoxyethylenether Triton X-100 oder Triton X-114 ist.

**6.** Verfahren gemäss Anspruch 4, worin der Polyoxyethylenether Triton X-100 ist.

**7.** Verfahren gemäss Anspruch 1 oder 2, worin das nichtionische Detergens ein Polyoxyethylen-Sorbitanester ist.

**8.** Verfahren gemäss Anspruch 7, worin der Polyoxyethylen-Sorbitanester Tween 20 bis Tween 80 ist.

**9.** Verfahren gemäss Anspruch 1 oder 2, worin gereinigtes OspA-Protein mit einer Konzentration von 1 mg/ml weniger als 1.000 ppm (0,1 %) nicht-ionisches Detergens enthält.

**10.** Verfahren gemäss Anspruch 9, worin das gereinigte OspA-Protein weniger als 100 ppm nicht-ionisches Detergens enthält.

**11.** Verfahren gemäss Anspruch 10, worin das gereinigte OspA-Protein weniger als 10 ppm nicht-ionisches Detergens enthält.

**12.** Verfahren gemäss Anspruch 1 oder 2, worin das zwitterionische Detergens ein N-Alkyl-N,N-dimethylammonium-1-propansulfonat ist.

**13.** Verfahren gemäss Anspruch 12, worin das N-Alkyl-N,N-dimethylammonium-1-propansulfonat SB8-SB18 ist.

**14.** Verfahren gemäss Anspruch 12, worin das N-Alkyl-N,N-dimethylammonium-1-propansulfonat SB12 ist.

**15.** Verfahren gemäss Anspruch 1 oder 2, worin gereinigtes OspA-Protein mit einer Konzentration von 1 mg/ml weniger als 1.000 ppb (1 ppm) des zwitterionischen Detergens enthält.

**16.** Verfahren gemäss Anspruch 15, worin das gereinigte OspA-Protein weniger als 100 ppb (0,1 ppm) des zwitterionischen Detergens enthält.

**17.** Verfahren gemäss Anspruch 16, worin das gereinigte OspA-Protein weniger als 10 ppb (0,01 ppm) des zwitterionischen Detergens enthält.

**18.** Verfahren gemäss Anspruch 1 oder 2, das zusätzlich die Sterilfiltration nach der Gelfiltration umfasst.

**19.** Impfstoff, der gereinigtes Lipoprotein OspA umfasst, das durch das Verfahren der Ansprüche 1 oder 2 erhältlich ist und das detergensfrei ist.

**20.** Impfstoff gemäss Anspruch 19, der zur menschlichen klinischen Verwendung geeignet ist.

**Revendications**

**1.** Procédé destiné à purifier de la lipoprotéine OspA de *Borrelia* comprenant:

(a) la lyse d'une cellule hôte exprimant la lipoprotéine OspA en présence d'un détergent zwitterionique pour former une solution impure contenant la lipoprotéine OspA;
(b) la clarification de la solution impure pour éliminer les débris de la cellule hôte;
(c) la mise en contact de la solution impure avec une résine échangeuse d'anions en présence d'un détergent non ionique;
(d) la récupération de l'effluent de ladite résine échangeuse d'anions et la mise en contact dudit effluent avec une résine échangeuse de cations; et
(e) l'élution de l'OspA suivie par une étape de filtration sur gel,

dans lequel la purification est réalisée sans chauffage.

**2.** Procédé destiné à purifier de la lipoprotéine OspA de *Borrelia* comprenant :

(a) la rupture de la cellule en présence d'un détergent zwitterionique pour former une solution impure contenant de l'OspA;

(b) la mise en contact de la solution impure avec un lit de résine fluidifié, ladite résine comprenant une résine échangeuse de cations;

(c) l'élution de l'OspA de ladite résine et la diafiltration de l'éluant par laquelle l'OspA est retenue;

(d) la mise en contact du rétentat avec une résine échangeuse d'anions en présence d'un détergent non ionique;

(e) la collecte de l'effluent de ladite résine échangeuse d'anions et la mise en contact dudit effluent fluide avec une résine échangeuse de cations; et

(f) l'élution d'OspA suivie d'une étape de filtration sur gel,

dans lequel la purification est réalisée sans chauffage.

3. Procédé selon la revendication 1 ou 2, dans lequel la clarification de la solution impure comprend une centrifugation.

4. Procédé selon la revendication 1 ou 2, dans lequel le détergent non ionique est un éther de polyoxyéthylène.

5. Procédé selon la revendication 4, dans lequel l'éther de polyoxyéthylène est le triton X-100 ou le triton X-114.

6. Procédé selon la revendication 4, dans lequel l'éther de polyoxyéthylène est le triton X-100.

7. Procédé selon la revendication 1 ou 2, dans lequel le détergent non ionique est un ester de polyoxyéthylènesorbitane.

8. Procédé selon la revendication 7, dans lequel l'ester de polyoxyéthylènesorbitane est le Tween 20 jusqu'au Tween 80.

9. Procédé selon la revendication 1 ou 2, dans lequel la protéine OspA purifiée à une concentration de 1 mg/ml contient moins de 1000 ppm (0,1%) de détergent non ionique.

10. Procédé selon la revendication 9, dans lequel la protéine OspA purifiée contient moins de 100 ppm de détergent non ionique.

11. Procédé selon la revendication 10, dans lequel la protéine OspA purifiée contient moins de 10 ppm de détergent non ionique.

12. Procédé selon la revendication 1 ou 2, dans lequel le détergent zwitterionique est un N-Alkyl-N,N-diméthylammonio-1-propanesulfonate.

13. Procédé selon la revendication 12, dans lequel le N-Alkyl-N,N-diméthylammonio-1-propanesulfonate est le SB8-SB18.

14. Procédé selon la revendication 12, dans lequel le N-Alkyl-N,N-diméthylammonio-1-propanesulfonate est le SB12.

15. Procédé selon la revendication 1 ou 2, dans lequel la protéine OspA purifiée à une concentration de 1 mg/ml contient moins de 1000 ppb (1 ppm) dudit détergent zwitterionique.

16. Procédé selon la revendication 15, dans lequel la protéine OspA purifiée contient moins de 100 ppb (0,1 ppm) dudit détergent zwitterionique.

17. Procédé selon la revendication 16, dans lequel la protéine OspA purifiée contient moins de 10 ppb (0,01 ppm) dudit détergent zwitterionique.

18. Procédé selon la revendication 1 ou 2, lequel comprend également une filtration stérile après la filtration sur gel.

19. Vaccin comprenant la lipoprotéine OspA purifiée obtenue par le procédé selon les revendications 1 ou 2, qui est exempt de détergent.

20. Vaccin selon la revendication 19, qui convient pour une utilisation en clinique humaine.

SB12  clearance study of 75L and 20L consistency lots

Legend:
- OPA113
- OPA114
- OPA115
- OPA013
- OPA014
- OPA015

X-axis (Steps): BZ5S, STC, RD, QFT, SPC

Y-axis: SB12 content (ppm)

**Fig. 1** Reduction of SB12 concentration by the different purification steps.

EP 1 080 109 B1